# EUROPEAN PATENT SPECIFICATION

(11) **EP 3 069 712 B1**
(45) Date of publication and mention of the grant of the patent: **23.09.2020**
(21) Application number: 14862046.1
(22) Date of filing: 17.11.2014
(51) Int. Cl.: A61K 9/10, A61K 31/343, A61K 47/10, A61K 47/18, A61K 47/24

(54) **TRANSDERMAL COLLOIDAL SOLUTION AGENT**
TRANSDERMALES KOLLOIDLÖSUNGSMITTEL
AGENT TRANSDERMIQUE EN SOLUTION COLLOÏDALE

(30) Priority: 17.11.2013 JP 2013237494; 24.02.2014 JP 2014033471
(43) Date of publication of application: 21.09.2016
(73) Proprietor: MEDRx Co., Ltd., Higashikagawa-shi Kagawa 769-2712 (JP)
(72) Inventor: YAMASAKI, Keiko, Higashikagawa-city Kagawa 769-2712 (JP)
(74) Representative: Miller Sturt Kenyon
(86) International application number: PCT/JP2014/080328
(87) International publication number: WO 2015/072564

(56) References cited:
- EP-A1- 2 471 539
- WO-A1-2010/126501
- WO-A1-2011/111384
- WO-A2-2010/036947
- JP-B2- 3 313 891
- JUN-BOM PARK ET AL: "Enhanced transdermal delivery and optimization of nano-liposome preparation using hydrophilic drug", JOURNAL OF KOREAN PHARMACEUTICAL SCIENCES, vol. 42, no. 2, 1 April 2012 (2012-04-01), pages 57-63, XP55309143, KOREA ISSN: 2093-5552, DOI: 10.1007/s40005-012-0009-4
- MANCONI ET AL.: 'Penetration enhancer containing vesicles as carriers for dermal delivery of tretinoin' INTERNATIONAL JOURNAL OF PHARMACEUTICS 2011, pages 37 - 46, XP055285752
- ZHAO ET AL.: 'Selection of high efficient transdermal lipid vesicle for curcumin skin delivery' INTERNATIONAL JOURNAL OF PHARMACEUTICS vol. 454, no. NEN 7, 2013, pages 302 - 309, XP055126433

## Description

### Technical Field

The present invention relates to a novel transdermal absorptive liquid formulation. Specifically, the present invention relates to an external preparation containing a medicament and phosphatidyl choline.

### Background Art

There are various methods to make a medicament permeate through the skin. An example of transdermal delivery is described in J-B Park et al "Enhanced transdermal delivery and optimization of nano-liposome preparation using hydrophilic drug" Journal of Korean Pharmaceutical Sciences 2012; 42 no. 2: 57-63. In general, the medicament is completely dissolved in a solvent, and the concentration gradient or effect of an absorption promoter ensure the permeability of the medicament. Alternatively, it is known that phosphatidyl choline is used to form a micelle, and to have a medicament permeate through the skin (Patent Document 1 and 2).

As an external liquid preparation containing phosphatidyl choline, a liquid preparation in which L-carnitine is contained to achieve an excellent absorbability of phosphatidyl choline (Patent Document 1), and an oil-in-water emulsion liquid preparation of ropinirole hydrochloride (Patent Document 2) are known.

The transdermal absorptive formulation may occasionally cause cutaneous irritation depending on a sort of the medicament. Therefore, suppressing the cutaneous irritation should be considered when the dosage form is designed. For example, cholesterol has been used as a cutaneous irritation suppressing agent of donepezil (Patent Document 3).

### Prior Art Documents

### Patent Documents

Patent Document 1: WO2011/024354
Patent Document 2: WO2011/111384
Patent Document 3: WO2011/136288

### Non Patent Documents

Non patent Document 1: P. Santos et al, Skin Pharmacol Physiol 2008;21:246-259
Non patent Document 2: Adenan Azeem et al, Drug Development and Industrial Pharmacy, 35: 252-547, 2009

### SUMMARY OF THE INVENTION

### Problem to be Solved by the Invention

An object of the present invention is to provide a transdermal absorptive liquid formulation which exhibits high transdermal permeability of a medicament, and whose cutaneous irritation is suppressed.

### Means for Solving the Problems

The present inventor found that the formulation in which a medicament and phosphatidyl choline are colloidally dispersed in Propylene glycol or a propylene glycol-containing solvent as particles having 50-500nm diameter exhibits extremely excellent transdermal permeability and maintains said excellent permeability compared to a solution in which a medicament is completely dissolved and exists in a molecular state. The inventor further found that the effects are doubled by adding an alkanolamine.

The subject matters of the present invention are as follows.
(1) A transdermal absorptive colloidal liquid formulation according to claim 1.
(2) The transdermal absorptive colloidal liquid formulation according to the above item (1), wherein the alkanolamine is triethanolamine.
(3) The transdermal absorptive colloidal liquid formulation according to the above item (1) or the above item (2), wherein the medicament contains a six-membered ring skeleton and a nitrogen containing group in a structure thereof.
(4) The transdermal absorptive colloidal liquid formulation according to any one of the above items (1) to (4), wherein the content of propylene glycol in the formulation is equal to or more than 60 w/w%.
(5) The transdermal absorptive colloidal liquid formulation according to any one of the above items (1) to (5), wherein the phosphatidyl choline is an unsaturated phosphatidyl choline.
(6) The transdermal absorptive colloidal liquid formulation according to any one of the above items (1) to (5), wherein the phosphatidyl choline is at least one selected from an egg yolk phosphatidyl choline and soybean phosphatidyl choline.

### EFFECTS OF THE INVENTION

The colloidal liquid formulation of the present invention has an extremely improved transdermal permeability and has achieved fast and persistent permeability by colloidally dispersing a medicament. Therefore, it is possible to achieve a high blood concentration same as when an oral preparation is administrated. Additionally, the cutaneous irritation can be markedly suppressed by administrating as the colloidal liquid preparation of the present invention even if the medicament is known to cause cutaneous irritation.

### BRIEF DESCRIPTION OF THE DRAWINGS

[Fig 1] Fig 1 shows the particle size distribution of the solution prepared in Reference Preparation Example.
[Fig 2] Fig 2 shows the particle size distribution of the colloidal liquid formulation containing galantamine hydrobromide prepared in Example 1c.
[Fig 3] Fig 3 is a graph showing the result of the blood concentration evaluation test of the colloidal liquid formulation containing galantamine prepared in Examples 1b and 1c.
[Fig 4] Fig 4 shows the particle size distribution of the colloidal liquid formulation containing ramelteon prepared in Example 2a.
[Fig 5] Fig 5 is a graph showing the result of the blood concentration evaluation test of the colloidal liquid formulation containing ramelteon prepared in Example 2a.
[Fig 6] Fig 6 shows the particle size distribution of the colloidal liquid formulation containing rasagiline prepared in Example 3.
[Fig 7] Fig 7 shows the particle size distribution of the colloidal liquid formulation containing indomethacin prepared in Example 5.
[Fig 8] Fig 8 is a graph showing the result of the blood concentration evaluation test of the colloidal liquid formulation containing donepezil prepared in Example 6.

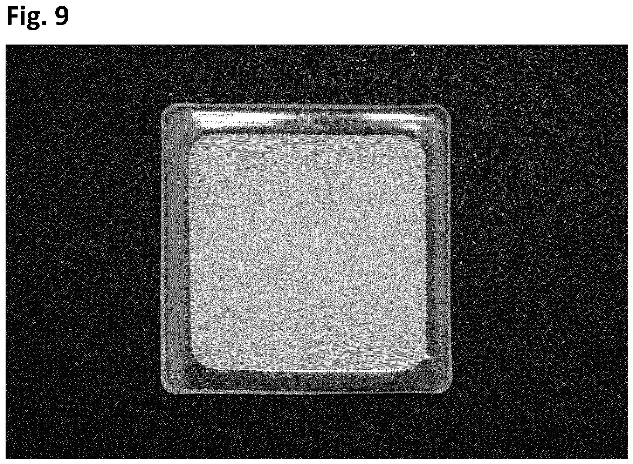

[Fig 9] Fig 9 is a photograph of the liquid type adhesive patch used in the blood concentration evaluation test.

### DETAILED DESCRIPTION OF THE INVENTION

The present invention relates to a transdermal absorptive colloidal liquid formulation as defined in claim 1, in which a medicament and phosphatidyl choline are dispersed in propylene glycol or a propylene glycol-containing solvent, and further comprising alkanolamine. The present invention also relates a method for preparing thereof. The term "propylene glycol-containing solvent" in this specification denotes propylene glycol to which a solubilizing agent such as water and/or a hydrophilic solvent is dissolved in and mixed as described below in detail.

The term "colloidal dispersion" in this description denotes a liquid exhibiting a clear Tyndall phenomenon by irradiation with red laser light. The average particle diameter of the dispersoid (colloidal particles) in the colloidal dispersions of the present invention is from 0.05 to 0.5 µm, preferably from 0.05 to 0.2µm. in addition, it has a mode of particle diameter around 0.1µm (from 0.04 to 0.15µm). This colloidal dispersion was stable and aggregation over time and the like were not observed.

Any of a neutral medicament, a basic medicament, and an acidic medicament can be utilize as the "medicament". The term "medicament" in this description includes its pharmaceutically acceptable salt. The "neutral medicament" means a non-ionic medicament such as a medicament having a hydroxyl group or an amide group. The "basic medicament" means a medicament having a primary, secondary or tertiary amino group and exhibiting basicity as whole compound. The "acidic medicament" means a medicament having carboxyl group or the like and exhibiting acidity as whole compound. The neutral medicament or the basic medicament is particularly preferred as the medicament, because they particularly exhibit the significant effects of the present invention such as excellent skin permeability and stability of the solution.

A compound having a six-membered ring skeleton and a nitrogen-containing group in its structure can be preferably utilized as the medicament. When the medicament has such a structure, a stable colloid to be formed, thus the effect of the present invention to improve the skin permeability is expressed significantly. Six-membered ring in the six-membered ring skeleton can be either homocyclic rings or heterocyclic ring. Said six-membered ring includes a ring having aromaticity, a saturated or unsaturated aliphatic ring which may have a bridged structure. Examples of the six-membered homocyclic rings are shown in (a) to (c), examples of the six-membered heterocyclic rings are shown in (d) to (f).

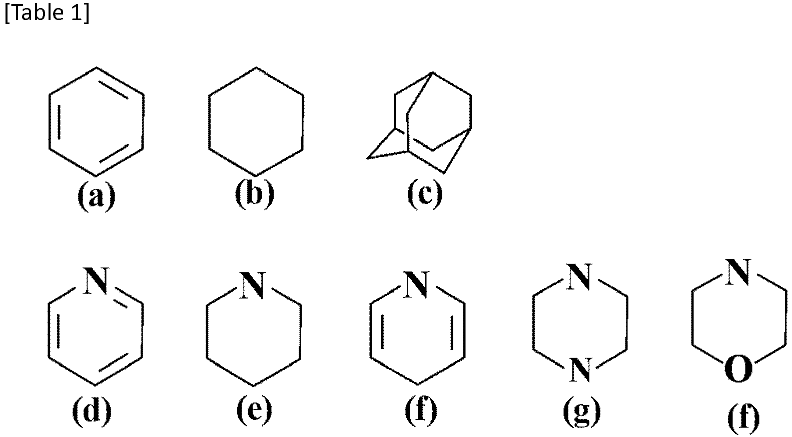

An amino group, an imino group, an amido group, a cyano group, a nitro group can be exemplified as the nitrogen-containing group. The nitrogen-containing group may be bound to the six-membered ring skeleton described above via an arbitrary organic group.

In case where the medicament contains six-membered ring having nitrogen such as pyridine, piperidine, and peperaine, said six-membered ring can also be recognized as the nitrogen-containing group. In other words, if nitrogen is contained as one of the constituent elements of the six-membered ring skeleton, the medicament is not required to contain other nitrogen-containing group as well.

It is preferred that the six-membered ring forms a structure represented by the following formula I-a or I-b, as the result of at least two adjacent carbon atoms have a substituent. 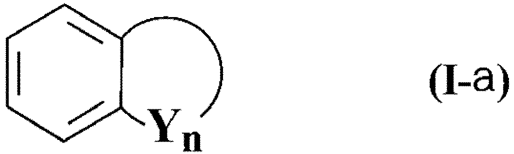 (in the formula, Y is same or different, and represent a carbon atom, a nitrogen atom, an oxygen atom, or a sulfur atom, n represents an integer of 3-5. Each atom represented by Y bind each other via a single bond or a double bond.) 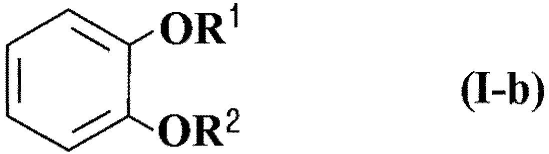 (in the formula, R¹ and R² are same or different, and represent an organic group which may bind to each other or to other substituents to form a ring or a hydrogen tom.)

The value of n is preferably 3 or 4, most preferably 3, in case where the medicament contains the structure represented by formula 1-1.

When n is 3, the structure represented by the formula I-a can be, for example, a condensed ring structure comprising a six-membered ring and a five-membered ring as shown in Table 2. Specifically, a homocyclic ring such as indane, indene and the like; a heterocyclic ring containing an oxygen atom such as benzofurin, isobenofurin, benzofuran, isobenzofuran, and the like; a heterocyclic ring containing two oxygen atoms such as benzodioxole and the like; a heterocyclic ring containing a nitrogen atom such as indoline, indole, isoindole, and the like; a heterocyclic ring containing two nitrogen atoms such as indazole, benzoimidazoline and the like; a heterocyclic ring containing a sulfur atom such as benzothiophene and the like; a heterocyclic ring containing an oxygen atom and a nitrogen atom such as benzoxazol, benzisoxazol, and the like; a heterocyclic ring containing a nitrogen atom and a sulfur atom such as benzothiadiazole and the like can be exemplified.

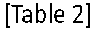

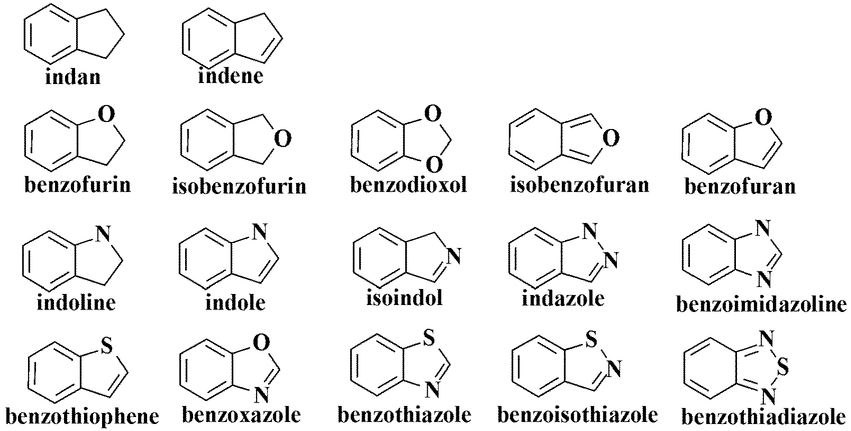

As the medicament containing the structure represented by the formula I-a and n is 3,a neutral medicament containing indane structure such as ramelteon, and the like; a basic medicament containing indane structure such as rasagiline, donepezil, and the like; a basic medicament containing isobenzofurin structure such as escitalopram, galantamine, and the like; a neutral medicament containing benzofurin structure such as ramelteon, and the like; a basic medicament containing benzofurin structure such as morphine, oxycodone, and the like; a basic medicament containing benzodioxole structure such as paroxetine, and the like; a basic medicament containing indoline structure such as ropinirole, and the like; an acidic medicament containing indole structure such as indomethacin, and the like; a basic medicament containing indole structure such as pergolide, bromocriptine, ondansetron, and the like; a basic medicament containing indazol structure such as granisetron, and the like; a basic medicament containing benzothiophene structure such as raloxifene, zileuton, and the like; a basic medicament containing benzoisothiazole structure such as lurasidone, and the like; a basic medicament containing benzothiadiazole structure such as tizanidine, and the like can be exemplified.

When n is 4, the structure represented by formula I-a has a structure in which two six-membered rings are condensed. Specifically, a naphthalene skeleton, a quinolone skeleton, an isoquinoline skeleton, a cinnolin skeleton, a quinazoline skeleton, a quinoxaline skeleton, a phthalazine skeleton, a chroman skeleton, and the like can be exemplified. As the medicament containing such a structure, apomorphine, morphine, oxycodone, pergolide, bromocriptine, propranolol, butorphanol, rotigotine, aripiprazole, doxazosin, quinapril, esuprone, procaterol, azelastine, chlorpromazine, and the like can be exemplified.

When n is 5, the structure represented by formula I-a is a condensed ring comprising a six-membered ring and a seven-membered ring. As the medicament containing such a structure, imipuran, flurazepam, diltiazem, ketotifen, and the like can be exemplified.

As the medicament containing the structure represented by the formula I-b, a basic medicament in which both R¹ and R² are hydrogen atoms such as apomorphine, dopamine, Isoprenaline, and the like; a basic medicament in which both R¹ and R² are aliphatic hydrocarbon groups such as donepezil, verapamil, oxypertine, and the like; a basic medicament in which R¹ is a hydrogen atom or an aliphatic group, R² is an aliphatic group which forms a ring together with at least two carbon atoms constituting the six-membered ring such as galantamine, morphine, oxycodone, and the like can be exemplified.

Additionally, as the medicament containing a six-membered ring skeleton and a nitrogen-containing group, a basic medicament containing pyridine structure such as nicametate, betahistine, and the like; a basic medicament containing piperidine skeleton such as difenidol, fentanyl, morphine, oxycodone, apomorphine, donepezil, methylphenidate, eperisone, pridinol, trihexyphenidyl, pyridoxal, and the like; a basic medicament containing dihydropyridine skeleton such as nicardipine, benidipine, efonidipine, and the like; a basic medicament containing piperidine structure such as flunarizine, and the like; a basic medicament containing morpholine skeleton such as timolol, and the like; a basic medicament containing adamantane skeleton such as amantadine, memantine, vildagliptin, and the like; a basic medicament containing benzene ring and a nitrogen-containing ring such as tolazoline, clemastine, rilmazafone, and the like; a basic medicament containing benzene ring and a secondary amino group such as bisoprolol, metoprolol, alprenolol, methamphetamine, tulobuterol, and the like; a neutral medicament containing benzene ring and a secondary amino group such as tolbutamide, glibenclamide, acetohexamide, and the like; a basic medicament containing benzene ring and a tertiary amino group such as oxybutynin, neostigmine, lidocaine, and the like; a basic medicament containing benzene ring and a primary amino group such as procaine, and the like can be exemplified.

A content of the medicament can be selected from the range of 0.1 to 10 w/w%, preferably the range of 0.5 to 5 w/w%.

Phosphatidyl choline is a collective term of a compound represented by formula (II), usually provided as mixture having different types and combinations of R¹ and R². 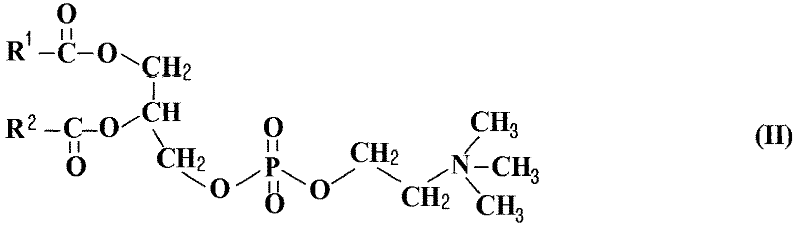 (in the formula , R¹ and R² are identical or different each other, and each is a C₁₂₋₂₂ hydrocarbon group)

An unsaturated phosphatidyl choline in which at least one of R¹ and R² is an unsaturated hydrocarbon group can be utilized in the present invention. Though it may be possible that a saturated hydrocarbon group such as palmityl group (16:0), stearyl group (18:0) is included as R¹ and R², the unsaturated phosphatidyl choline which can be utilize in the present invention contains less than 80% of the saturated hydrocarbon group, preferably less than 70% of the saturated hydrocarbon group, more preferably less than 60% of the saturated hydrocarbon group, most preferably less than 50% of the saturated hydrocarbon group. Palmitoyl group (16:1), oleyl group (18:1), linoleyl group (18:2), linolenyl group (18:3) can be exemplified as the unsaturated hydrocarbon group. It is preferred that the content of the unsaturated hydrocarbon group having 18 carbon atoms such as oleyl group, linoleyl group, linolenyl group, and the like is equal to or more than 20%, more preferably equal to or more than 30%, particularly preferably equal to or more than 40%. With the use of the unsaturated phosphatidyl choline, a stable colloidal dispersion exhibiting excellent transdermal permeability can be prepared.

A highly purified phosphatidyl choline which can be utilize in the present invention is a naturally derived phosphatidyl choline such as a soybean lecithin, an egg yolk lecithin, and whose content of the phosphatidyl choline is equal to or more than 95%. It is not preferable to use a chemically and/or biologically modified phosphatidyl choline such as a hydrogenated phosphatidyl choline obtained from hydrotreatment or a lysophosphatidyl choline is used, because stable colloidal dispersion may not be obtained. However, of the chemically or biologically modified phosphatidyl choline, a highly purified phosphatidyl choline having a high unsaturation degree (for example, the iodine value is 20 or more, and content of lysolecithin is less than 10%) such as a partial hydrogenated product of a naturally derived phosphatidyl choline can be utilized as the "unsaturated phosphatidyl choline" of the present invention. If the colloid is not formed, enhancing effects to the transdermal absorbability of the medicament is limited.

The concentration of phosphatidyl choline is selected from the range of 0.1 to 5w/w%, preferably 0.2 to 3.0w/w%, more preferably 0.3 to 2.0w/w%, particularly preferably 0.3 to 1.5w/w%. It is unfavorable that the concentration of phosphatidyl choline is less than 0.1w/w%, because stable colloidal dispersion may not be formed. Adding phosphatidyl choline excess to 5w/w% does not cause the improvement of transdermal permeability depending on the increase in the phosphatidyl choline concentration.

The liquid formulation of the present invention in which a medicament and phosphatidyl choline are colloidally dispersed in propylene glycol or propylene glycol-containing solvent exhibits excellent preservation stability and transdermal permeability. Moreover, transdermal permeability of the medicament is improved spectacularly by further containing an alkanolamine as an absorption promoter. Primary, secondary or tertiary alkanolamine having 2 to 12 carbon atoms can be used as the alkanolamine. Among these, secondary or tertiary alkanolamine are preferred, tertiary alkanol amine is particularly preferred. Specifically, diethanolamine, triethanolamine, diisopropanolamine, and triisopropanolamine can be exemplified. Triethanolamine is particularly preferred, since transdermal permeability promoting effect is excellent.

The concentration of alkanolamine is selected from the range of 0.01 to 10w/w%, depending on the properties of the medicament. In most of the medicament, the transdermal permeability promoting effect is remarkably exhibited in the range of 1 to 8w/w%, particularly 2 to 5 w/w%. However, in some case, it is undesirable to add alkanolamine exceeding 1 w/w%. Donepezil hydrochloride described below is an example of the case.

Property of the liquid formulation of the present invention will depend on both property of the dissolved medicament or its salt and additive amount of the alkanolamine. Though preferred property of the liquid formulation of the present invention is weak alkaline, some medicaments are unstable under alkaline condition. Therefore, the additive amount of alkanolamine can be appropriately adjusted. For example, in case of donepezil hydrochloride, desirable additive amount of triethanolamine is 0.01 to 0.5w/w%, because adding it in excess to 1% causes precipitation and aggregation of the crystal. As just described, the pH of the liquid formulation can be adjusted appropriately with the additive amount of alkanolamine depending on stability of the medicament.

"Propylene glycol" used in the present invention is not particularly limited, and commercially available ones can be used. The colloidal dispersion of the present invention can be prepared by mixing the medicament of the dissolved state in propylene glycol with phosphatidyl choline of the dissolved state in propylene glycol as described below in detail. Some salts of the basic medicament have low solubility in propylene glycol (for example, less than 1w/w%), thus the medicament can't be dissolved in propylene glycol at a desired quantity. In such occasion, a solubilizing agent such as water and polyethylene glycol is added in propylene glycol to from propylene glycol-containing solvent. Then, the medicament of dissolved state in propylene glycol can be prepared by dissolving the medicament to said propylene glycol-containing solvent. The additive amount of the solubilizing agent can be selected from a range in which a content of propylene glycol in propylene glycol-containing solvent is equal to or more than 50w/w%, preferably equal to or more than 60w/w%, specifically preferably equal to or more than 65w/w%. When additive amount of solubilizing agent is too much, thus propylene glycol content is reduced, stable colloid which exhibit an excellent transdermal permeability of the medicament may not be formed. It is not preferable.

Propylene glycol-containing solvent can further include a hydrophilic solvent which is miscible with propylene glycol if needed. Specifically, a polyol such as glycerin, 1,3-butanediol can exemplified. The content of the hydrophilic solvent is less than 10w/w% of propylene glycol-containing solution.

Though a little amount of polyol such as glycerin or butanediol can be added to propylene glycol, it is not suitable for the colloidal liquid of the present invention to use said polyol instead of propylene glycol. For example, when phosphatidyl choline is added to glycerin, white turbidity and gathering of phosphatidyl choline toward surface of the liquid are observed, phosphatidyl choline tends to from an association colloid by itself. Additionally, it tends to gelate after medicament is added. On the other hand, phosphatidyl choline is completely dissolved into 1,3-butandiol as molecule similar to propylene glycol. However, after adding the medicament, it aggregation and precipitation tend to occur in short period unlike in the case of propylene glycol.

The liquid formulation of the present invention does not contain any hydrophobic solvent. The term "hydrophobic solvent" in this specification denotes an oleaginous solvent which doesn't dissolve in propylene glycol in arbitrary ratio. Specifically, hydrocarbons such as liquid paraffin, squalene and the like; higher fatty acids such as oleic acid, lauric acid, myristic acid, palmitic acid, stearic acid, and the like; higher alcohol such as cetyl alcohol, stearyl alcohol, myristyl alcohol, and the like; a fatty acid esters such as isopropyl myristate, isopropyl palmitate, butyl stearate, and the like; vegetable oils such as olive oil, camellia oil, jojoba oil, and the like cam be exemplified.

The colloidal liquid formulation of the present invention contains propylene glycol at a concentration equal to or more than 60w/w%, and consists only components which are soluble in propylene glycol or propylene glycol-containing solvent.

Various additives which are conventionally used in an external preparation or a cosmetics can be added to the colloidal liquid formulation of the present invention if necessary. As the possible additives, perfumes, antioxidant agent, antiseptic agent, coloring agent, buffering agent, pH adjusting agent, and the like can be exemplified. As the perfumes, ethanol, orange essence, and the like are exemplified. As the antioxidant agent, tocopherol acetate, edetate sodium, erythorbic acid, 1,3-butylene glycol, sodium metabisulfite, and the like can be exemplified. As the antiseptic agent, sorbic acid, taurine, and the like can be exemplified. As the pH adjusting agent, organic acids such as citric acid, acetic acid, acidum tartaricum, and the like; inorganic acids such as phosphoric acid, hydrochloride, and the like can be exemplified. Moreover, an ultraviolet absorber and an antibacterial agent can be added depending on purposes.

The colloidal dispersion of the present invention can be prepared by mixing the medicament of dissolved state in propylene glycol or propylene glycol-containing solvent with phosphatidyl choline of the dissolving state in propylene glycol or propylene glycol-containing solvent. When anyone of the medicament or phosphatidyl choline is dissolved, it becomes a true solution, and particles whose diameter is equal to or more than 10nm is not observed. Both the medicament solution and phosphatidyl choline solution does not show Tyndall phenomenon. However, when the medicament of dissolved state is mixed with phosphatidyl choline of dissolved state, it becomes a colloidal dispersion showing Tyndall phenomenon, and the mode particle size thereof is observed around 100nm.

As a method for mixing the medicament of dissolved state and phosphatidyl choline of dissolved state, following methods are exemplified, but are not particularly limited. That is, a method of a solution (solution I) in which medicament is dissolved in propylene glycol or propylene glycol-containing solvent and the other solution (solution II) in which phosphatidyl choline is dissolved in propylene glycol or propylene glycol-containing solvent are prepared respectively, thus mixing said solution I with said solution II; a method of adding phosphatidyl choline into said solution I and mixing it; a method of adding the medicament into said solution II and mixing it; a method of adding phosphatidyl choline and the medicament at the same time into propylene glycol or propylene glycol-containing solvent. The method in which solution I and solution II are prepared respectively and then they are mixed together is excellent in the point that it can be reliably confirmed phosphatidyl choline and the medicament are in their dissolved state. By mixing and stirring the medicament of the dissolved state with phosphatidyl choline of the dissolved state, a stable colloidal dispersing liquid having an average particle diameter of 50 to 500 nm can be obtained.

Alkanolamine and other additives can be added at any time.

A method for adapting the liquid formulation to the skin is not particularly limited, and a method of coating with, or splaying; a method of attaching a suitable carrier carrying the liquid formulation on the skin can be exemplified. Among them, a method of attaching a foamed matrix carrying the liquid formulation therein is preferable, because it has good handling property and is easy to adjust the dosage. The "foamed matrix" is a porous body obtained by forming resins with a physical or chemical procedure. Specifically, polyurethane foam can be exemplified. The "polyurethane foam" is obtained by forming polyol and polyisocyanate with polymerizing adding a bloating agent, a foam stabilizer, a catalyst, and a coloring agent. Polyurethane foam is abbreviated to urethane foam, and is broadly classified into the following three groups by foaming process. That is, a "soft urethane foam" having interconnected cells, is soft and possesses restorability, a "rigid urethane foam" having closed pores, is hard and has no resilience, a "semi-rigid urethane form" having intermediate properties. The rigid urethane foam of discontinuity type is preferable because leakage is small when it carries medicament solution.

### Examples

Hereinafter, the present invention is explained in detail with examples. The present invention is not limited in any way by these examples

### [Reference Preparation Example]

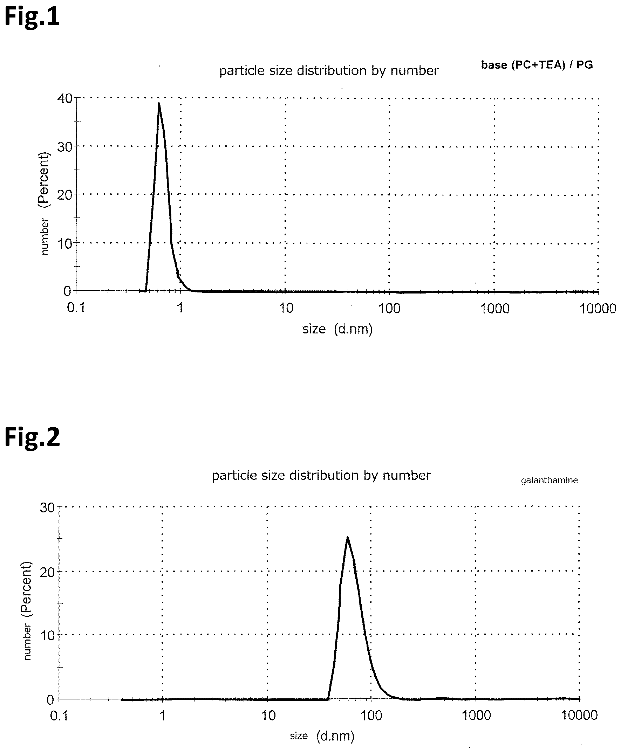

Phosphatidyl choline (0.5 part by weight) was dissolved to propylene glycol (94.5 part by weight), further triethanolamine (5 part by weight) was added and stirred, to obtain a clear liquid. A red laser was irradiated to the liquid, but Tyndall phenomenon was not observed. Though the particle size distribution was measured by Zeta Sizer Nano (made by Malvern Instruments, inc.), any distribution having particle size of more than was not observed. The measurement result of the particle size distribution is shown in Fig 1.

### [preparation of a liquid formulation containing galantamine]

### (Examples 1a to 1c) and (Comparative Examples 1a to 1c)

The liquid formulations as the composition shown in Table 3 are prepared. The obtained clear solution is irradiated with a red laser beam, to observe the presence or absence of Tyndall Phenomenon. Furthermore, transdermal permeability of galantamine of the obtained liquid formulation was evaluated with the use of Franz cell. Skin used for the test was 5 weeks old hairless rats (male) abdominal excised skin, the receptor solution was (water: ethanol =9 :1) solution. The results are shown in Table 3.

**[Table 3]**

| | Ex.1a | Ex.1b | Com.1a | Com.1b | Ex.1c | Com.1c |
|---|---|---|---|---|---|---|
| galantamine | 5.0 | 5.0 | 5.0 | 5.0 | - | - |
| galantamine HBr | - | - | - | - | 4.0 | 4.0 |
| PG | 94.5 | 89.5 | 90.0 | 89.5 | 70.5 | - |
| PEG | - | - | - | - | - | 61.5 |
| purified water | - | - | - | - | 20.0 | 30.0 |
| PC | 0.5 | 0.5 | - | - | 0.5 | 0.5 |
| lysolecithin | - | - | - | 0.5 | - | - |
| TEA | - | 5.0 | 5.0 | 5.0 | 4.0 | 4.0 |
| total | 100 | 100 | 100 | 100 | 100 | 100 |
| Presence or absence of Tyndall phenomenon | presence | presence | absence | absence | presence | absence |
| Comulative skin permeation amount at 6hours (µg/cm²) | 208 | 359 | 15 | 37 | 226 | 18 |

| | | | | | | |
|---|---|---|---|---|---|---|
| PG : propylene glycol PEG : polyethylene glycol PC : phosphatidyl choline TEA : triethanolamine | | | | | | |

Liquid formulation of Example 1a containing phosphatidyl choline showed excellent skin permeability compared to liquid formulation of Comparative Example 1a containing no phosphatidyl choline. Liquid formulation of Example 1b further containing ethanolamine showed superior skin permeability to liquid formulation of Example 1a. Liquid formulation of Comparative Example 1b containing lysolecithin instead of phosphatidyl choline didn't show Tyndall phenomenon, and didn't exhibit improvement of skin permeability. Galanthamine hydrobromide has low solubility to propylene glycol. Therefore, it was dissolved to propylene glycol-containing solvent in which purified water was added as solubilizing agent, thus prepared colloidal liquid with the same procedure as free galanthamine (Example 1c). The obtained liquid formulation exhibited excellent skin permeability. Liquid formulation of Comparative Example 1c containing polyethylene glycol instead of propylene glycol didn't show Tyndall phenomenon, and didn't exhibit improvement of skin permeability.

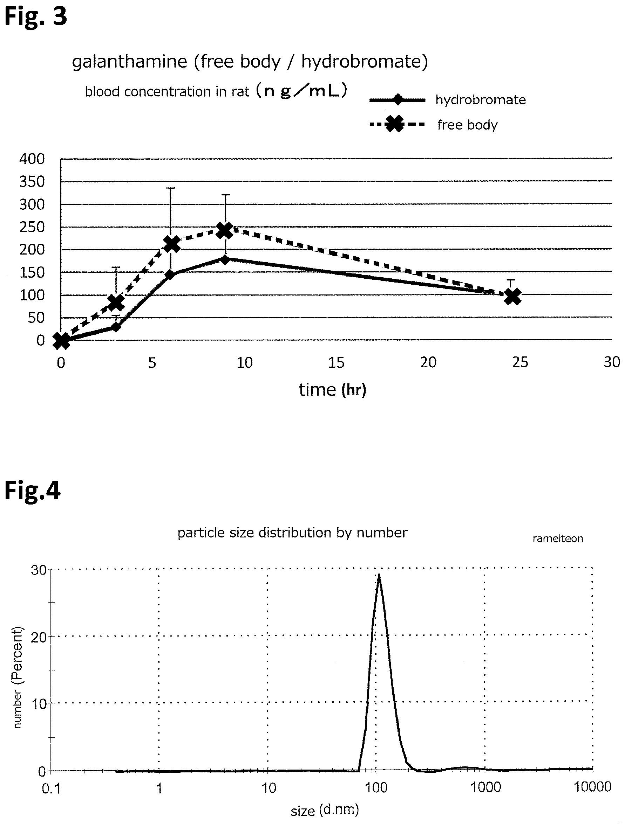

Particle size distribution of liquid formulation of Example 1c was measured by Zeta Sizer Nano (made by Malvern Instruments, inc.). Mode of the particle size was observed at around 70nm. The measurement result is shown in Fig 2. Liquid formulation of Example 1b and Example 1c (0.08g) was impregnated into urethane foam (area:2cm² thickness:0.5mm bulk density:0.2g/3cm³) to prepare liquid type adhesive patch. Prepared liquid type adhesive patch were adhered to back of rats (5 weeks old, male) and transition of blood concentration in rats was evaluated according to conventional method. The result is shown in Figure 3. Both the liquid formulation of Example 1c containing free galanthamine and the liquid formulation of Example 1c containing hydrobromate exhibit excellent transdermal absorbability, and there was no difference in the transition of blood concentration.

### [preparation of liquid formulation containing ramelteon]

### (Example 2a) and (Comparative Examples 2a to 2e)

The liquid formulations as the composition (w/w%) shown in Table 4 are prepared. Skin permeability of Ramelteon was evaluated by Franz cell for obtained liquid formulation. The results are shown in Table 4. The particle size distribution was measured by Zeta Sizer Nano (made by Malvern Instruments, inc.) for liquid formulation of 2a. Mode of the particle size was observed at around 110nm. The result is shown in Fig 4. Skin used in the Franz cell test was abdominal excised skin of 5 weeks old hairless rats(male), and the receptor solution was (water: ethanol =9:1) solution.

**[Table 4]**

| | | Ex. 2a | Com. 2a | Com. 2b | Com. 2c | Com. 2d | Com. 2e |
|---|---|---|---|---|---|---|---|
| Ramelteon | | 5.0 | 5.0 | 5.0 | 10.0 | 5.0 | 5.0 |
| PG | | 89.5 | 89.5 | 89.5 | 90.0 | 90.0 | 90.0 |
| PC | | 0.5 | - | - | - | - | - |
| Tween80 | | - | 0.5 | - | - | - | - |
| HC040 | | - | - | 0.5 | - | - | - |
| TEA | | 5.0 | 5.0 | 5.0 | - | - | 5.0 |
| Palmitic acid | | - | - | - | - | 5.0 | - |
| total | | 100 | 100 | 100 | 100 | 100 | 100 |
| Presence or absence of Tyndall phenomenon | | presence | absence | absence | absence | absence | absence |
| Comulative skin permeation amount (µg/cm²) | at 6 hours | 72 | 23 | 19 | - | 7 | 13 |
| | at 24 hours | 1324 | 211 | 197 | 12 | 91 | 112 |

| | | | | | | | |
|---|---|---|---|---|---|---|---|
| PG : propylene glycol PC : phosphatidyl choline POC40 : Polyoxyethylene hardened castor oil TEA: triethanolamine | | | | | | | |

All the liquid formulations of Comparative Examples 2a to 2e didn't form colloidal liquid, and had poor skin permeability. Comparative Examples 2a and 2b contain Tween80 and HCO40 instead of phosphatidyl choline. They are same as phosphatidyl choline at the point having surface activity. However, both the Comparative Example 2a and 2b didn't form colloidal liquid and didn't show significant improvement in skin permeability. Therefore, phosphatidyl choline possesses a peculiar transdermal absorption promoting activity different from any other usual surfactant.

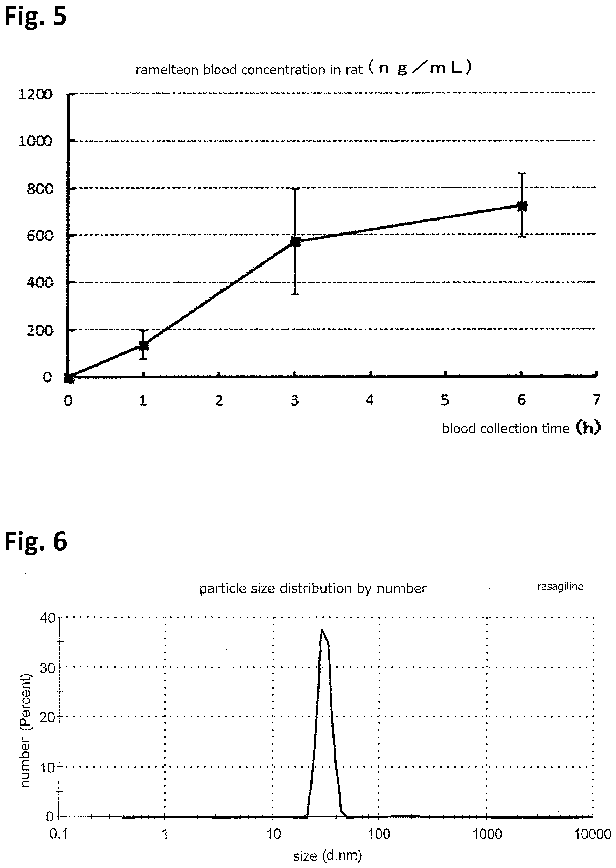

Liquid formulation of Example 2a (0.3g) was impregnated into urethane foam (area:9cm² thickness:0.5mm bulk density:0.2g/3cm³) to prepare liquid type adhesive patch. Prepared liquid type adhesive patch were adhered to back of rats (5 weeks old, male) and transition of blood concentration in rats was evaluated according to conventional method. The result is shown in Figure 5. The adhesive patches which had been adhered to rat for 6hors were collected to measure amount of ramelteon remaining in the adhesive patch and surface of the skin. Residual ration of ramelteon was about 65%. Namely, about 35% of ramelteon contained in the adhesive patch was transferred into the blood. The discharge rate of ramelteon was about 5.5mg/sheet.

### [Examination of content of phosphatidyl choline and absorption promoter]

The liquid formulations as the composition (w/w%) shown in Table 5 are prepared. Skin permeability of was evaluated by Franz cell for each liquid formulations. The results are shown in Table 5. Skin used in the Franz cell test was abdominal excised skin of 5 weeks old hairless rats(male), and the receptor solution was (water: ethanol =9:1) solution.

**[Table 5]**

| | | Ex. 2a | Ex. 2a | Ex. 2c | Ex. 2d | Ex. 2e | Ex. 2f |
|---|---|---|---|---|---|---|---|
| Ramelteon | | 5.0 | 5.0 | 5.0 | 5.0 | 5.0 | 5.0 |
| PG | | 89.75 | 89.5 | 87.0 | 87.0 | 89.5 | 89.5 |
| PC | | 0.25 | 0.5 | 3.0 | 3.0 | 0.5 | 0.5 |
| TEA | | 5.0 | 5.0 | 5.0 | - | - | - |
| DEA | | - | - | - | - | 5.0 | - |
| DIPA | | - | - | - | - | - | 5.0 |
| Palmitic acid | | - | - | - | 5.0 | - | - |
| Presence or absence of Tyndall phenomenon | | presence | presence | presence | presence | presence | presence |
| total | | 100 | 100 | 100 | 100 | 100 | 100 |
| Comulative skin permeation amount (µg/cm²) | at 6 hours | 26 | 72 | 88 | 17 | 18 | 32 |
| | at 24 hours | 826 | 1324 | 1153 | 239 | - | - |

| | | | | | | | |
|---|---|---|---|---|---|---|---|
| PG : propylene glycol PC : phosphatidyl choline TEA : triethanolamine DEA : diethanolamine DIPA: diisopropanolamine | | | | | | | |

The skin permeation amount increased depending on content of phosphatidyl choline. However, a marked increase in skin permeability was not observed with the addition of phosphatidyl choline exceed 0.5w/w%. Therefore, the content of Phosphatidyl choline is usually selected from in the range of 0.1 to 5 w/w%, preferably 0.3 to 2.0 w/w%. Triethanolamine exhibits significant skin permeation accelerated effect in shorter period compare to the other absorption promoters such as diethanolamine, diisopropanolamine, and the like.

### [Examination of content of absorption promoter]

### (Examples 2g to 2j)

The liquid formulations as the composition (w/w%) shown in Table 6 are prepared. Skin permeability of was evaluated by Franz cell for each liquid formulations. The results are shown in Table 6. Skin used in the Franz cell test was abdominal excised skin of 5 weeks old hairless rats(male), and the receptor solution was (water: ethanol =9:1) solution.

**[Table 6]**

| | | Ex. 2g | Ex. 2h | Ex. 2i | Ex. 2a | Ex. 2j |
|---|---|---|---|---|---|---|
| Ramelteon | | 5.0 | 5.0 | 5.09 | 5.0 | 5.0 |
| PG | | 94.0 | 93.5 | 91.5 | 89.5 | 54.5 |
| PC | | 0.5 | 0.5 | 0.5 | 0.5 | 0.5 |
| TEA | | 0.5 | 1.0 | 3.0 | 5.0 | 10.0 |
| total | | 100 | 100 | 100 | 100 | 100 |
| Comulative skin permeation amount (µg/cm²) | at 6 hours | 13 | 20 | 55 | 72 | 58 |
| | at 24 hours | 438 | 720 | 1146 | 1324 | 1103 |

| | | | | | | |
|---|---|---|---|---|---|---|
| PG : propylene glycol PC : phosphatidyl choline TEA: triethanolamine | | | | | | |

The skin permeation amount increased depending on the amount of triethanolamine. The content of triethanolamine can be selected from 0.01 to 10 w/w%. In order to obtain a sufficient absorption promoting effect, it is preferably 1 to 8 w/w%, particularly preferably 2.5 to 5.5 w/w%.

### [preparation of liquid formulations containing various medicaments]

### (Examples 3 to 7b) and (Comparative Examples 3 to 7)

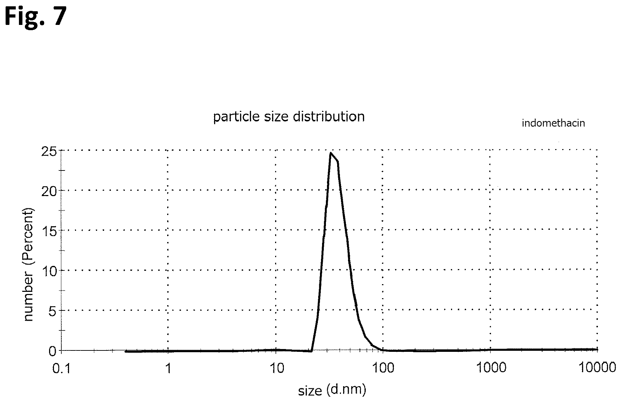

The liquid formulations as the composition (w/w%) shown in Table 7 and Table 8 are prepared. Skin permeability of was evaluated by Franz cell for each liquid formulations. The results are shown in Table 7 and Table 8. The particle size distribution was measured by Zeta Sizer Nano(made by Malvern Instruments, inc.). The results are shown in Figure 6 and Figure7. Skin used in the Franz cell test was abdominal excised skin of 5 weeks old hairless rats(male), and the receptor solution was (water: ethanol =9:1) solution.

**[Table 7]**

| | | Ex. 3 | Com. 3 | Ex. 4 | Com. 4 | Ex. 5 | Com. 5 | Ex. 6 | Com. 6 |
|---|---|---|---|---|---|---|---|---|---|
| Rasagiline mesylate | | 6.25 | 6.25 | - | - | - | - | - | - |
| Escitalopram maleate | | - | - | 2.0 | 2.0 | - | - | - | - |
| Indomethacin | | - | - | - | - | 0.5 | 0.5 | - | - |
| Donepezil hydrochloride | | - | - | - | - | - | - | 5.0 | 5.0 |
| PG | | 88.25 | 88.75 | 92.5 | 93.0 | 99.0 | 99.5 | 78.3 | 79.3 |
| Purified water | | - | - | - | - | - | - | 15.0 | 15.0 |
| PC | | 0.5 | - | 0.5 | - | 0.5 | - | 1.0 | - |
| TEA | | 5.0 | 5.0 | 5.0 | 5.0 | - | - | 0.5 | 0.5 |
| Sodium pyrosulfite | | - | - | - | - | - | - | 0.2 | 0.2 |
| total | | 100 | 100 | 100 | 100 | 100 | 100 | 100 | 100 |
| Presence or absence of Tyndall phenomenon | | presen ce | absenc e | presen ce | absenc e | prese nce | absen ce | prese nce | absenc e |
| Comulative skin permeation amount (µg/cm²) | at 2 hours | 93 | 24 | - | - | - | - | - | - |
| | At 6 hours | - | - | 45 | 0 | - | - | 77 | 3 |
| | at 24 hours | - | - | - | - | 282 | 57 | - | - |

| | | | | | | | | | |
|---|---|---|---|---|---|---|---|---|---|
| PG : propylene glycol PC : phosphatidyl choline TEA: triethanolamine | | | | | | | | | |

**[Table 8]**

| | | Ex. 7a | Ex.7b | Com.7 |
|---|---|---|---|---|
| Apomorphine HCL | | 3.0 | 3.0 | 3.0 |
| PG | | 96.3 | 95.8 | 96.8 |
| PC | | 0.5 | 0.5 | - |
| TEA | | - | 0.5 | - |
| Sodium pyrosulfite | | 0.2 | 0.2 | 0.2 |
| total | | 100 | 100 | 100 |
| Presence or absence of Tyndall phenomenon | | presenc e | presenc e | absence |
| Comulative skin permeation amount (µg/cm²) | at 6 hours | 20 | 50 | 0 |
| | at 24 hours | 459 | 1428 | 0 |

| | | | | |
|---|---|---|---|---|
| PG : propylene glycol PC: phosphatidyl choline TEA: triethanolamine | | | | |

At all the medicament, colloidal liquid formulations containing phosphatidyl choline showed marked improvement in skin permeation amount compared to liquid formulation containing no phosphatidyl choline. Addition of triethanolamine caused further dramatic increase in skin permeation amount. The colloidal liquid formulation of the present invention exhibits excellent transdermal permeation for any of basic medicament acidic medicament, and neutral medicament.

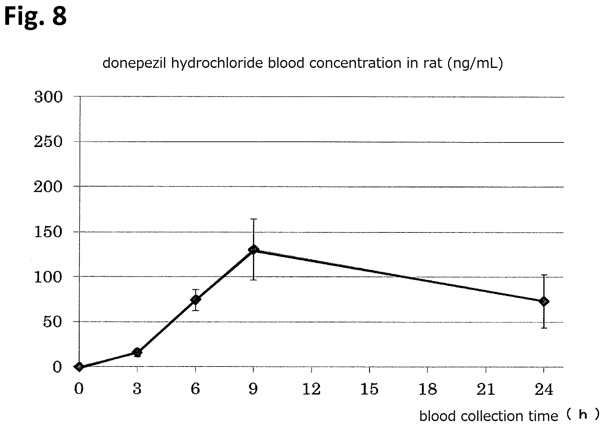

Liquid formulation of Example 6 (0.18g) was impregnated into urethane foam (area:6cm² thickness:0.5mm bulk density:0.2g/cm³) to prepare liquid type adhesive patch. Prepared liquid type adhesive patch were adhered to back of rats(5 weeks old, male) and transition of blood concentration in rats was evaluated according to conventional method. The result is shown in Figure 8. Though skin irritation caused by donepezil has been reported, any indication such as erythema was not observed at all on the skin of rats after the adhesive patch were peeled off. It is presumed to be due to skin protective effect of phosphatidyl choline.

### Industrial Applicability

The colloidal liquid formulation of the present invention can be used as a liquid preparation for administering a variety of medicament transdermally.

## Claims

1. A transdermal absorptive colloidal liquid formulation wherein a medicament or a salt thereof is colloidally dispersed in propylene glycol or a propylene glycol-containing solvent in the presence of phosphatidyl choline wherein:
the formulation further comprises from 0.01 to 10 w/w% an alkanolamine; and
the content of the phosphatidyl choline is from 0.1 to 5 w/w%; and
the content of propylene glycol in the propylene glycol-containing solvent is equal to or more than 50 w/w%.

2. The transdermal absorptive colloidal liquid formulation according to claim 1, wherein the alkanolamine is triethanolamine.

3. The transdermal absorptive colloidal liquid formulation according to claim 1 or claim 2, wherein the medicament contains a six-membered ring skeleton and a nitrogen containing group in a structure thereof.

4. The transdermal absorptive colloidal liquid formulation according to any one of claims 1 to 3, wherein the content of propylene glycol in the formulation is equal to or more than 60 w/w%.

5. The transdermal absorptive colloidal liquid formulation according to any one of claims 1 to 4, wherein the phosphatidyl choline is an unsaturated phosphatidyl choline.

6. The transdermal absorptive colloidal liquid formulation according to any one of claims 1 to 5, wherein the phosphatidyl choline is at least one selected from an egg yolk phosphatidyl choline and soybean phosphatidyl choline.

## Patentansprüche

1. Transdermale, absorptionsfähige, kolloidale, flüssige Formulierung, wobei ein Medikament oder ein Salz davon kolloidal in Propylenglykol oder einem Propylenglykol enthaltenden Lösungsmittel in Gegenwart von Phosphatidylcholin dispergiert ist, wobei:
die Formulierung ferner 0,01 bis 10 Gew./Gew.-% Alkanolamin umfasst; und
der Gehalt des Phosphatidylcholins 0,1 bis 5 Gew./Gew.-% beträgt; und
der Gehalt von Propylenglykol in dem Propylenglykol enthaltenden Lösungsmittel gleich 50 Gew./Gew.-% oder höher ist.

2. Transdermale, absorptionsfähige, kolloidale, flüssige Formulierung nach Anspruch 1, wobei das Alkanolamin Triethanolamin ist.

3. Transdermale, absorptionsfähige, kolloidale, flüssige Formulierung nach Anspruch 1 oder Anspruch 2, wobei das Medikament ein sechsgliedriges Ringskelett und eine stickstoffhaltige Gruppe in einer Struktur davon enthält.

4. Transdermale, absorptionsfähige, kolloidale, flüssige Formulierung nach einem der Ansprüche 1 bis 3, wobei der Gehalt von Propylenglykol in der Formulierung gleich 60 Gew./Gew.-% oder höher Ist.

5. Transdermale, absorptionsfähige, kolloidale, flüssige Formulierung nach einem der Ansprüche 1 bis 4, wobei das Phosphatidylcholin ein ungesättigtes Phosphatidylcholin ist.

6. Transdermale, absorptionsfähige, kolloidale, flüssige Formulierung nach einem der Ansprüche 1 bis 5, wobei das Phosphatidylcholin mindestens eines ist ausgewählt unter einem Eigelbphosphatidylcholin und einem Sojabohnenphosphatidylcholin.

## Revendications

1. Formulation liquide colloïdale absorbante transdermique dans laquelle un médicament ou un de ses sels est dispersé de manière colloïdale dans du propylène glycol ou un solvant contenant du propylène glycol en présence de phosphatidyl choline :
la formulation comprenant en outre de 0,01 à 10 p/% en poids d'une alcanolamine ; et
la teneur de la phosphatidyl choline étant de 0,1 à 5 p/% en poids ; et
la teneur de propylène glycol dans le solvant contenant du propylène glycol étant supérieure ou égale à 50 p/% en poids.

2. Formulation liquide colloïdale absorbante transdermique selon la revendication 1, dans laquelle l'alcanolamine est la triéthanolamine.

3. Formulation liquide colloïdale absorbante transdermique selon la revendication 1 ou la revendication 2, dans laquelle le médicament contient un squelette de cycle à six chaînons et un groupe contenant de l'azote dans une de de ses structures.

4. Formulation liquide colloïdale absorbante transdermique selon l'une quelconque des revendications 1 à 3, dans laquelle la teneur de propylène glycol dans la formulation est supérieure ou égale à 60 p/% en poids.

5. Formulation liquide colloïdale absorbante transdermique selon l'une quelconque des revendications 1 à 4, dans laquelle la phosphatidyl choline est une phosphatidyl choline insaturée.

6. Formulation liquide colloïdale absorbante transdermique selon l'une quelconque des revendications 1 à 5, dans laquelle la phosphatidyl choline est au moins une choisie parmi une phosphatidyl choline de jaune d'œuf et une phosphatidyl choline de soja.
